# EUROPÄISCHE PATENTSCHRIFT

(11) **EP 0 996 607 B1**
(45) Veröffentlichungstag und Bekanntmachung des Hinweises auf die Patenterteilung: **14.11.2001**
(21) Anmeldenummer: 98940135.1
(22) Anmeldetag: 03.07.1998
(51) Int. Cl.: C07C 11/09, C07C 7/148, C07C 41/06

(54) **VERFAHREN UND VORRICHTUNG ZUR GEWINNUNG VON ISOBUTEN AUS EINEM KOHLENWASSERSTOFFGEMISCH**
METHOD AND DEVICE FOR OBTAINING ISOBUTENES FROM CONJUGATED HYDROCARBONS
PROCEDE ET DISPOSITIF POUR L'OBTENTION D'ISOBUTENES A PARTIR D'UN MELANGE D'HYDROCARBURE

(30) Priorität: 04.07.1997 DE 19728732
(43) Veröffentlichungstag der Anmeldung: 03.05.2000
(73) Patentinhaber: BASF AKTIENGESELLSCHAFT, 67056 Ludwigshafen (DE)
(72) Erfinder: BESSLING, Bernd, D-67269 Grünstadt (DE); KNAB, Jean, Werner, D-67117 Limburgerhof (DE); BROX, Wolfgang, D-69118 Heidelberg (DE); LOHE, Bernd, D-69115 Heidelberg (DE)
(74) Vertreter: Isenbruck, Günter
(86) Internationale Anmeldenummer: EP9804135
(87) Internationale Veröffentlichungsnummer: WO9901411

(56) Entgegenhaltungen:
- EP-A- 0 015 513
- EP-A- 0 022 510
- WO-A-97/35823
- US-A- 4 510 336

## Beschreibung

Die Erfindung betrifft ein Verfahren und eine Vorrichtung zur Abtrennung von Isobuten aus C₄-Kohlenwasserstoffgemischen über eine Etherzwischenstufe.
Der dabei verwendete, durch Extraktivdestillation meist bereits schon weitgehend von Butadien befreite C₄-Schnitt, enthält in der Regel neben Isobuten hauptsächlich 1-Buten, 2-Buten (cis oder trans) und n-Butan. Diese C₄-Kohlenwasserstoffgemische lassen sich durch einfache fraktionierte Destillation wegen zu geringer Siedepunktsdifferenz ihrer Komponenten nicht mehr wirtschaftlich auftrennen. Es kommen daher leistungsfähigere, selektivere, physikalische und zusätzlich auch chemische Trennverfahren zum Einsatz.

Letztere werden insbesondere auch für die Abtrennung des Isobutens angewendet, da dieses sich unter anderem durch seine höhere Reaktivität von den übrigen C₄-Komponenten unterscheidet. Das der Erfindung zugrundeliegende chemische Trennverfahren beruht auf der Umsetzung von Isobuten mit einem primären C₃- oder C₄-Alkanol in Gegenwart eines Katalysators zu dem entsprechenden tertiären Ether. Nach dessen destillativer Abtrennung vom restlichen C₄-Kohlenwasserstoffgemisch und nachfolgender Rückspaltung zu Isobuten und den entsprechenden primären C₃- oder C₄-Alkanol wird nach einem weiteren destillativen Trennschritt das Isobuten als Kopfprodukt erhalten. An dieser Stelle werden einige Begriffe definiert, die in den nachfolgenden Ausführungen verwendet werden:

Das Wort "Einbauten" wird als allgemeiner Begriff für Böden, Füllkörper und Packungen genutzt. Einbauten, die zusätzlich die Fixierung heterogener Katalysatoren ermöglichen (siehe beispielsweise EP-B-0 396 650, EP-B-0 008 860), werden im folgenden als reaktive Einbauten (Reaktionsböden, reaktive Füllkörper, reaktive Packung) bezeichnet. Einbauten zur ausschließlich destillativen Zwecken werden als konventionell bezeichnet.

Bei einem bekannten Verfahren zur Gewinnung von Isobuten aus C₄-Kohlenwasserstoffgemischen wird darin enthaltenes Isobuten mit primärem C₃- oder C₄-Alkanol zu dem betreffenden tertiären Ether umgesetzt (EP-B-0 015 513 und EP-B-0 003 305). Die Reaktion wird mit Ionenaustauscherharzen katalysiert. Um einen hinreichenden Umsatz zu erzielen, erfolgt die Umsetzung in einer Kaskade aus drei Reaktoren, zwischen denen gekühlt wird. Im ersten Reaktor wird aufgrund von kinetischen Aspekten bei höherer Temperatur verethert als in dem zweiten bzw. dritten Reaktor, bei denen die Temperatur so eingestellt wird, daß diese sich günstig auf das chemische Gleichgewicht der exothermen Veretherung auswirkt. Um zufriedenstellende Umsätze zu erreichen, muß außerdem mit einem deutlichen Überschuß an primärem Alkanol gearbeitet werden. Bei der Verwendung von Isobutanol beträgt das molare Einsatzverhältnis von letzterem zu Isobuten etwa 1,7:1 (gebildeter Ether: Isobutyltertiärbutylether (IBTBE)). In einer Destillationskolonne wird anschließend der entsprechende tertiäre Ether von dem restlichen C₄-Kohlenwasserstoffgemisch abgetrennt. Nach nachfolgender thermischer, katalytischer Spaltung des Ethers wird Isobuten als Kopfprodukt einer weiteren Destillation erhalten.

Der Erfindung lag die Aufgabe zugrunde, daß vorstehend beschriebene Verfahren zur Gewinnung von Isobuten aus C₄-Kohlenwasserstoffgemischen so zu verbessern, daß
1. der apparative Aufwand reduziert wird;
2. höhere Umsätze erzielt werden, so daß das molare Einsatzverhältnis von dem primären C₃- bzw. C₄-Alkanol zu Isobuten reduziert werden kann.

Die Lösung dieser Aufgabe geht aus von dem Verfahren zur Gewinnung von Isobuten aus einem Kohlenwasserstoffgemisch durch
a) Zusammenführung des C₄-Kohlenwasserstoffgemischs mit einem primären C₃- oder C₄-Alkanol,
b) Umsetzung des Isobutens im C₄-Kohlenwasserstoffgemisch mit dem primären C₃- oder C₄-Alkanol in Gegenwart eines heterogenen Katalysators zu dem entsprechenden tertiären Ether des Isobutens,
c) Trennung des anfallenden Reaktionsgemischs in die niedriger siedenden unveretherten C₄-Kohlenwasserstoffe und in den höhersiedenden tertiären Ether des Isobutens mit Hilfe einer Destillationskolonne, wobei die C₄-Kohlenwasserstoffe über Kopf abgezogen werden und der im Sumpf anfallende tertiäre Ether des Isobutens in einen Reaktor überführt wird,
d) Spaltung des Ethers in Isobuten und in das entsprechende primäre C₃-oder C₄-Alkanol,
e) Destillation dieses Gemisches aus d) in einer weiteren Destillationskolonne und Abzug des Isobuten als Kopfprodukt.

Das erfindungsgemäße Verfahren ist dadurch gekennzeichnet, daß die Stufe
a) in einer Zone durchgeführt wird, die reaktive Einbauten enthält, in denen der Katalysator zur Durchführung der Stufe b) angeordnet ist, daß die Zone in einer Destillationskolonne integriert ist, daß in dieser Zone eine Reaktivdestillation abläuft und daß das C₃- oder C₄-Alkanol oberhalb und das C₄-Kohlenwasserstoffgemisch unterhalb der Zone der Destillationskolonne zugeführt werden.

Erfindungsgemäß wird auch eine Vorrichtung zur Durchführung des oben genannten Verfahren bereitgestellt, wobei diese folgende Einrichtungen enthält:
i) eine Destillationskolonne mit reaktiven und konventionellen Einbauten,
ii) zwei Phasentrenner,
iii) ein Katalysatorbett,
iv) eine weitere Destillationskolonne und

Verbindungsleitungen zwischen den Einrichtungen i) bis iv).

Die erfinderische Lösung, das zugrundeliegende chemische Trennverfahren mit Hilfe einer Reaktivdestillation durchführen zu können, beruht vermutlich darauf, daß unter gleichen Bedingungen eine Reaktion (Isobuten reagiert mit einem primären Alkanol) und eine Destillation durchgeführt werden können. Voraussetzung ist, daß das Produkt einen höheren Siedepunkt aufweist als die Edukte, daß die Edukte verschiedene Siedepunkte haben (Voraussetzung für Gegenstromführung der Edukte) und daß die Siedepunktsdifferenz der beiden Edukte nicht zu hoch ist (ausreichende Verweilzeit der Edukte ist in der Zone notwendig, in der die Reaktion stattfindet). Außerdem muß die Reaktion unter den Bedingungen der Destillation mit einem hohen Umsatz durchführbar sein, die Selektivität hoch sein, und die ensprechende Veretherung muß reversibel sein.

Eine genauere Bezeichnung für Zone als Ort der Veretherung wäre bei dem vorliegende Verfahren das Wort Reaktivdestillationszone. Diese ist im mittleren Teil der Destillationskolonne angeordnet und enthält erfindungsgemäß reaktive Einbauten, wobei in den Bereichen oberhalb und unterhalb dieser Zone reine Destillationen ablaufen. Für diese Bereiche sind konventionelle Einbauten vorgesehen. Als reaktiver Einbau kann ein Katalysator auf den entsprechenden Verweilzeitböden, z.B. in deren Ablauf vorliegen, oder im Falle von Packungen in diese taschenartig eingeschlossen sein. Füllkörper können entsprechend beschichtet sein. Als Katalysator können geeignete bekannte Katalysatoren verwendet werden. Hierzu gehören beispielsweise Ionenaustauscherharze in der [H⁺]-Form (z.B. Levatit® der Bayer AG Leverkusen). Je nach Temperaturbeständigkeit der Katalysatoren kann die Reaktionstemperatur und damit der Druck variieren. Der entsprechende Anstieg der Reaktionsgeschwindigkeit erlaubt eine kompaktere Gestaltung der Kolonne. Die Temperaturen sind so zu wählen, daß der Katalysator nicht geschädigt wird. Gleichzeitig ist es wünschenswert, die Kondensationstemperaturen deutlich über der Umgebungstemperatur zu halten. Die Zusammenhänge zwischen Konzentration, Druck und Temperaturen für ein gegebenes Stoffsystem sind an sich bekannt.

Für das System Isobuten/primäres C₃- beziehungsweise C₄-Alkanol/betreffender tertiärer Ether folgen für eine thermische Belastungsgrenze des Katalysators von zum Beispiel 150°C damit ein möglicher Druckbereich von 3 bis 6 bar und Temperaturen von 25 bis 190°C im Kolonneninneren. Zu bevorzugen ist ein Bereich von etwa 5 bar. Die Temperatur in der Reaktivdestillationszone der Kolonne beträgt dann 35°C bis 190°C, vorzugsweise 50°C bis 160°C. Höhere Temperaturen können nicht nur schädlich für den Katalysator sein, sondern begünstigen auch Nebenreaktionen wie die Eliminierung von Wasser beim Alkanol, die Oligomerisierung von Isobuten oder die Veretherung zweier Alkanole.

Durch die Abhängigkeit der Reaktionsgeschwindigkeit von der Systemtemperatur bestimmt sich das erforderliche Katalysatorvolumen. Ein wesentlicher Vorteii der beschriebenen Reaktivdestillation, bedingt durch die Gegenstromführung der Edukte, sind die durch die günstigere Einstellung des chemischen Gleichtgewichtes hervorgerufenen hohen Umsätze bei der Veretherung.

Bei gleichem molaren Einsatzverhältnis von primärem Alkohol zu Isobuten werden höhere Umsätze erzielt als in dem durch EP-B-0 015 513 beziehungsweise EP-B-0 003 305 beschriebenen Verfahren (Kaskade aus drei Reaktoren). Andererseits kann aufgrund des hohen Umsatzes das molare Einsatzverhältnis von primärem Alkohol zu Isobuten deutlich reduziert werden. Letzteres liegt für das beschriebene Verfahren vorzugsweise bei 1,1:1. Ein geringerer Alkanolgehalt in der Reaktivdestillationszone bewirkt eine Reduzierung des aus dem Sumpf abzuführenden "Restalkanols". Damit ist einerseits eine Verringerung der Kreislaufströme verbunden und andererseits wird das chemische Gleichgewicht der Etherspaltung günstig beeinflußt. Das beschriebene Verfahren eignet sich insbesondere aufgrund der hohen Umsätze bei der Veretherung zur Aufbereitung von C₄-Kohlenwasserstoffgemischen mit besonders geringem Isobutengehalt (Isobuten ist für bestimmte Folgereaktionen schädlich). Hierfür wird zweckmäßigerweise ein höherer Eduktanteil des primären Alkohols gewählt. Das molare Einsatzverhältnis vom primärem Alkohol zu Isobuten beträgt allgemein etwa 1,7:1 bis 1,0:1.

Aufgrund der Verringerung des apparativen Aufwandes, erfindungsgemäß gelöst durch die Integration der Reaktionszone (in EP-B-0 015 513 bzw. EP-B-0 003 305 Kaskade aus drei Reaktoren für die Veretherung) in die Destillationskolonne, werden nicht nur Materialkosten beim Anlagenbau gespart, sondern es wird andererseits auch die für das Verfahren aufzuwendende Energie vermindert. Die Reaktionswärme der exothermen Veretherung wird vorteilhafterweise außerdem direkt für die Destillation genutzt.

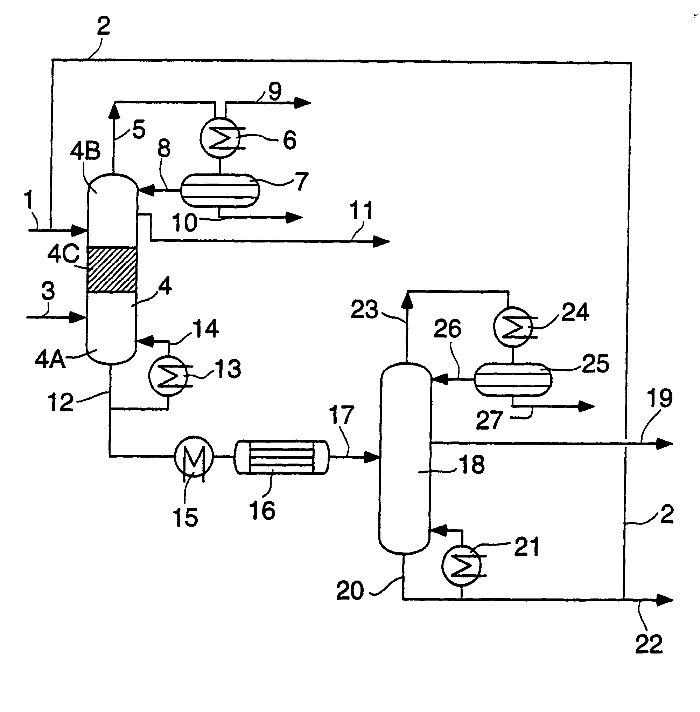

Weitere Einzelheiten und Vorteile der Erfindung können der folgenden Beschreibung des in der Zeichnung schematisch dargestellten Ausführungsbeispiels des erfindungsgemäßen Verfahrens entnommen werden.

Hier werden dem oberen Teil einer Destillationskolonne frisches Isobutanol durch eine Leitung 1 sowie im Kreislauf geführtes Isobutanol durch eine Leitung 2 zugeführt. Getrennt hiervon wird dem unteren Teil der Destillationskolonne 4 ein C₄-Kohlenwasserstoffgemisch durch eine Leitung 3 zugeführt. Das durch Leitung 3 zugeführte, Isobuten enthaltende C₄-Kohlenwasserstoffgemisch kann z.B. aus der thermischen Spaltung von Erdölprodukten stammen. Es enthält dann neben Isobuten z.B. noch Isobutan, n-Butan, 1-Buten, trans-2-Buten, cis-2-Buten und restliches Butadien. Die Destillationskolonne 4 ist mit übereinander angeordneten Einbauten versehen, die drei getrennte Bereiche bilden. Der untere Bereich konventioneller Einbauten 4A bildet einen Abtriebsteil. Der obere Bereich konventioneller Einbauten 4B bildet einen Verstärkungsteil. Die in der Mitte liegende Zone reaktiver Einbauten 4C ist ein Reaktivdestillationsteil. Die in dieser Zone 4C liegenden reaktiven Packungen sind mit einem heterogenen Katalysator versehen. Das durch die Leitung 3 zugeführte C₄-Gasgemisch wird unterhalb dieses Zone 4C zugeführt, das Isobutanol wird durch die Leitung 1 oberhalb dieser Zone 4C eingeführt.

Eine getrennte Zuführung der beiden Edukte bewirkt eine Gegenstromführung. In dem Verstärkungsteil 4B wird das schwerflüchtige Isobutanol von dem C₄-Rest getrennt. Im Abtriebsteil 4A wird das IBTBE von einem Isobutanolazeotrop getrennt und als Sumpfprodukt gewonnen. Die Gegenstromführung der Edukte und das destillative Abtrennen der Produkte aus der Zone 4C ermöglicht hohe Umsätze. Dabei verläßt nur ein geringer Anteil des Isobutanols die Kolonne 4. Das Einsatzverhältnis von Isobutanol zu Isobuten liegt in der Regel bei 1,1:1.

Vom Kopf der Kolonne 4 wird durch Leitung 5 ein von Isobuten befreiter C₄-Schnitt abgezogen und im Wärmetauscher 6 kondensiert. Die Kondensate werden dann dem Phasentrenner 7 zugeführt. Die dort anfallende, im wesentlichen aus C₄-Schnitt bestehende organische Phase wird durch Leitung 8 in den Kopf der Kolonne 4 zurückgeführt. Eventuelle Restfeuchte kann als wäßrige Phase durch Leitung 10 abgeführt werden. Vom oberen Teil des Wärmetauschers 6 wird Abgas durch Leitung 9 abgeführt. Durch die Leitung 11 werden C₄-Kohlenwasserstoffe abgezogen, die kein Isobuten mehr enthalten. Dieses Gasgemisch kann in anschließenden Verfahrensschritten beispielsweise zu Oktenen, Nonanol oder Weichmachern verarbeitet werden.

Vom Abtriebsteil 4A der Destillationskolonne 4 wird durch die Leitung 12 das gebildete IBTBE abgezogen, ein Teil davon wird in einem Wärmetauscher 13 erhitzt und über Leitung 14 in den Sumpf der Kolonne 4 zurückgeführt. Das anfallende IBTBE wird noch gespalten und destillativ aufbereitet. Das Isobutanol wird zur Veretherungskolonne zurückgeführt. Dies geschieht in der im folgenden beschriebenen Weise.

Das anfallende IBTBE wird über einen Wärmetauscher 15 auf die für die nachfolgende Reaktion erforderliche Temperatur eingestellt und einem Katalysatorbett 16 zugeleitet. In diesem wird das IBTBE zu Isobuten und Isobutanol gespalten und durch Leitung 17 der weiteren Destillationskolonne 18 zugeführt. In dieser mit konventionellen Einbauten versehenen Kolonne 18 wird das Zielprodukt Isobuten destillativ abgetrennt. Durch die Leitung 19 wird reines Isobuten abgezogen. Das im wesentlichen aus Isobutanol sowie nicht umgesetztem IBTBE bestehende Sumpfprodukt der Kolonne 18 verläßt diese durch Leitung 20. Ein Teil davon wird nach Erhitzung durch Wärmetauscher 21 wieder in die Kolonne 18 zurückgeführt. Der Hauptteil dieses Isobutanols gelangt durch Leitung 2 als Kreislauf-Isobutanol zurück zur Kolonne 4. Ein kleiner Teil wird zur Ausschleusung von schwersiedenden Verunreinigungen über die Leitung 22 abgetrennt. Das vom Kopf der Kolonne 18 durch Leitung 23 abgezogene Gasgemisch wird im Wärmetauscher 24 kondensiert und im nachgeschalteten Phasentrenner 25 aufgetrennt in ein Kondensat aus Kohlenwasserstoffen, das durch die Leitung 26 in die Kolonne 18 zurückgeführt wird, sowie Wasser, das durch Leitung 27 abgeführt wird.

Im folgenden wird die Veretherung von Isobuten mit Isobutanol mit der erfindungsgemäßen Reaktivdestillation zusätzlich anhand eines Beispiels beschrieben.

Die Laborkolonne 4 besteht z.B. aus einer Kolonne mit 0,055 m Durchmesser. Verstärkungsteil 4B und Abtriebsteil 4A (untere Reaktionszone) wurden durch je zwei konventionelle Elemente strukturierter Packungen (300 m²/m³) à 1 m realisiert. Den Reaktivteil 4C bildete eine reaktive Packung, die mit einem Ionenaustauscherharz (Handelsbezeichnung Lewatit® 2631 der Bayer AG Leverkusen) versehen ist; getrennt in 4 Einbauelemente ä 1 m, jeweils mit Sammlern und Verteilern. Der Katalysatoranteil betrug ca. 30 Vol%.

Die Zuführung von 2,5 kg/h Isobutanol erfolgte oberhalb der Zone 4C, die C₄-Kohlenwasserstoffe in einer Menge von 6,5 kg/h mit 25 Gew.-% Isobuten wurde unter der Zone 4C zugeführt. IBTBE wurde als Sumpfprodukt flüssig durch Leitung 12 abgezogen. Die nicht reagierten Leichtsieder (C₄) wurden kondensiert und als Destillat abgezogen, das Rücklaufverhältnis betrug etwa 2.

Der Betriebsdruck betrug 5 bar, um Kondensationstemperaturen über Raumtemperatur zu ermöglichen. Die Temperaturen an den reaktiven Einbauten führen dabei noch nicht zur Schädigung des Katalysators. Bei einem Druck von 5 bar stellten sich Sumpf- und Kopftemperatur zu 165 bzw. 47 °C ein. Im Reaktionsteil und destillativen Verstärkungsteil wurden die Temperaturen (ungefähr 65 °C) von den C₄-Komponenten bestimmt.

Das Isobuten konnte zu mehr als 97% mit Isobutanol umgesetzt werden.

Es wurde mit einem molaren Verhältnis Isobutanol/Isobuten von 1,1/1 gefahren. Dies liegt deutlich unter dem Wert von 1,7/1 und mehr der konventionellen, sequentiellen Reaktor-Kolonnen-Schaltung.

Das Kopfprodukt enthielt keine meßbaren Anteile Isobutanol oder IBTBE und < 1,5 Gew.-% Isobuten. Im Sumpfprodukt fand sich neben IBTBE Spuren von Isobuten (0,3 Gew.-%), das Überschußbutanol (ca. 10 Gew.-%) und 1 Gew.-% andere Butylether. Direkt unterhalb der Reaktivdestillationszone fanden sich etwa 50 Gew.-% IBTBE und 5 Gew.-% Isobutanol.

## Patentansprüche

1. Verfahren zur Gewinnung von Isobuten aus einem Kohlenwasserstoffgemisch durch
a) Zusammenführung des C₄-Kohlenwasserstoffgemischs mit einem primären C₃- oder C₄-Alkanol,
b) Umsetzung des Isobutens im C₄-Kohlenwasserstoffgemisch mit dem primären C₃- oder C₄-Alkanol in Gegenwart eines heterogenen Katalysators zu dem entsprechenden tertiären Ether des Isobutens,
c) Trennung des anfallenden Reaktionsgemischs in die niedriger siedenden unveretherten C₄-Kohlenwasserstoffe und in den höhersiedenden tertiären Ether des Isobutens mit Hilfe einer Destillationskolonne, wobei die C₄-Kohlenwasserstoffe über Kopf abgezogen werden und der im Sumpf anfallende tertiäre Ether des Isobutens in einen Reaktor überführt wird,
d) Spaltung dieses Ethers in Isobuten und in das entsprechende primäre C₃- oder C₄-Alkanol,
e) Destillation dieses Gemisches aus d) in einer weiteren Destillationskolonne und Abzug des Isobutens als Kopfprodukt,
**dadurch gekennzeichnet, daß** die Stufe a) in einer Zone (4C) durchgeführt wird, die reaktive Einbauten enthält, in denen der Katalysator zur Durchführung der Stufe b) angeordnet ist, **daß** die Zone (4C) in einer Destillationskolonne (4) integriert ist, **daß** in dieser Zone (4C) eine Reaktivdestillation abläuft und **daß** das C₃- oder C₄-Alkanol oberhalb und das C₄-Kohlenwasserstoffgemisch unterhalb der Zone (4C) der Destillationskolonne (4) zugeführt werden.

2. Verfahren nach Anspruch 1, **dadurch gekennzeichnet, daß** die Destillationskolonne (4) in getrennte Teilbereiche mit reaktiven und konventionellen Einbauten unterteilt ist und die Zone (4C) im mittleren Bereich angeordnet ist.

3. Verfahren nach Anspruch 1 oder 2, **dadurch gekennzeichnet, daß** das molare Einsatzverhältnis des Alkanols zu Isobuten bei 1,7:1 bis 1,0:1, bevorzugt bei 1,1:1 liegt.

4. Verfahren nach einem der Ansprüche 1 bis 3, **dadurch gekennzeichnet, daß** die Temperatur in der Zone (4C) 35°C bis 190°C, vorzugsweise 50°C bis 160°C beträgt.

5. Verfahren nach einem der Ansprüche 1 bis 4, **dadurch gekennzeichnet, daß** der Druck in der Zone (4C) 3 bis 6 bar, bevorzugt etwa 5 bar beträgt.

6. Verfahren nach einem der Ansprüche 1 bis 5, **dadurch gekennzeichnet, daß** als primäres Alkanol Isobutanol eingesetzt wird.

7. Verfahren nach einem der Ansprüche 1 bis 6, **dadurch gekennzeichnet, daß** als heterogener Katalysator ein Ionenaustauscher eingesetzt wird.

8. Vorrichtung zur Durchführung des Verfahrens nach einem der Ansprüche 1 bis 7 enthaltend folgende Einrichtungen:
i) eine Destillationskolonne (4) mit reaktiven und konventionellen Einbauten
ii) zwei Phasentrenner (7) und (25),
iii) ein Katalysatorbett (16),
iv) eine weitere Destillationskolonne (18) und
Verbindungsleitungen zwischen den Einrichtungen i) bis iv).

## Claims

1. A process for isolating isobutene from a hydrocarbon mixture by
a) combining the C₄-hydrocarbon mixture with a primary C₃- or C₄-alkanol;
b) reacting the isobutene in the C₄-hydrocarbon mixture with the primary C₃- or C₄-alkanol in the presence of a heterogeneous catalyst to give the corresponding tertiary ether of isobutene,
c) separating the resultant reaction mixture into the relatively low-boiling, unetherified C₄-hydrocarbons and the relatively higher-boiling tertiary ether of isobutene with the aid of a distillation column, where the C₄-hydro-carbons are taken off at the top, and the tertiary ether of isobutene obtained at the bottom is transferred into a reactor,
d) cleaving this ether into isobutene and the corresponding primary C₃- or C₄-alkanol,
e) distilling this mixture from d) in a further distillation column, and taking off the isobutene as the top product,
which comprises carrying out step a) in a zone (4C) containing reactive internals and containing the catalyst for carrying out step b) arranged in such a way that the zone (4C) is integrated into a distillation column (4), that a reactive distillation takes place in this zone (4C), and that the C₃- or C₄-alkanol is fed to the distillation column above the zone (4C) and the C₄-hydrocarbon mixture is fed to the distillation column (4) below the zone (4C).

2. A process as claimed in claim 1, wherein the distillation column (4) is divided into separate zones containing reactive and conventional internals, and the zone (4C) is arranged in the central region.

3. A process as claimed in claim 1 or 2, wherein the molar starting ratio between the alkanol and isobutene is from 1.7:1 to 1.0:1, and is preferably 1.1:1.

4. A process as claimed in any one of claims 1 to 3, wherein the temperature in zone (4C) is from 35°C to 190°C, preferably from 50°C to 160°C.

5. A process as claimed in any one of claims 1 to 4, wherein the pressure in zone (4C) is from 3 to 6 bar, and is preferably about 5 bar.

6. A process as claimed in any one of claims 1 to 5, wherein the primary alkanol employed is isobutanol.

7. A process as claimed in any one of claims 1 to 6, wherein the heterogeneous catalyst employed is an ion exchanger.

8. An apparatus for carrying out a process as claimed in any one of claims 1 to 7, comprising the following devices:
i) a distillation column (4) containing reactive and conventional internals
ii) two phase separators (7) and (25),
iii) a catalyst bed (16),
iv) a further distillation column (18), and
connecting lines between devices i) to iv).

## Revendications

1. Procédé pour isoler l'isobutène contenu dans un mélange d'hydrocarbures par
a) jonction du mélange d'hydrocarbures en C4 et d'un alcanol primaire en C3 ou C4,
b) réaction de l'isobutène contenu dans le mélange d'hydrocarbures en C4 avec l'alcanol primaire en C3 ou C4 en présence d'un catalyseur hétérogène, donnant l'éther tertiaire correspondant de l'isobutène,
c) séparation du mélange de réaction ainsi obtenu en les hydrocarbures en C4 non éthérifiés qui bouillent le plus bas et l'éther tertiaire de l'isobutène qui bout le plus haut à l'aide d'une colonne de distillation d'où les hydrocarbures en C4 sont évacués en tête et l'éther tertiaire de l'isobutène, resté dans le culot, est transféré dans un réacteur,
d) scission de cet éther en isobutène et l'alcanol primaire correspondant en C3 ou C4,
e) distillation du mélange formé en d) dans une autre colonne à distiller d'où l'isobutène est évacué en produit de tête,
**caractérisé par le fait que** l'étape a) est réalisée dans une zone (4C) contenant des éléments internes réactifs dans lesquels est disposé le catalyseur prévu pour la mise en oeuvre de l'étape b), la zone (4C) est intégrée à une colonne de distillation (4), dans cette zone (4C) il se produit une distillation réactive et l'alcanol en C3 ou C4 est introduit au-dessus et le mélange d'hydrocarbures en C4 au-dessous de la zone (4C) de la colonne de distillation (4).

2. Procédé selon la revendication 1, **caractérisé par le fait que** la colonne de distillation (4) est subdivisée en compartiments séparés avec des éléments internes réactifs et éléments internes classiques et la zone (4C) se trouve dans la région médiane.

3. Procédé selon la revendication 1 ou 2, **caractérisé par le fait que** l'alcanol est mis en oeuvre à un rapport molaire de 1,7 : 1 à 1,0 : 1, de préférence de 1,1 : 1 avec l'isobutène.

4. Procédé selon l'une des revendications 1 à 3, **caractérisé par le fait que** la température dans la zone (4C) va de 35 à 190°C, de préférence de 50 à 160°C.

5. Procédé selon l'une des revendications 1 à 4, **caractérisé par le fait que** la pression dans la zone (4C) va de 3 à 6 bar, et est de préférence d'environ 5 bar.

6. Procédé selon l'une des revendications 1 à 5, **caractérisé par le fait que** l'alcanol primaire mis en oeuvre est l'isobutanol.

7. Procédé selon une des revendications 1 à 6, **caractérisé par le fait que** le catalyseur hétérogène mis en oeuvre est un échangeur d'ions.

8. Appareillage pour la mise en oeuvre du procédé selon une des revendications 1 à 7, contenant les dispositifs suivants :
i) une colonne de distillation (4) avec éléments internes réactifs et classiques,
ii) deux séparateurs de phases (7) et (25),
iii) un lit de catalyseur (16),
iv) une autre colonne de distillation (18) et
des conduits de liaison entre les dispositifs i) à iv).
